(19) Europäisches Patentamt

European Patent Office

Office européen des brevets

(11) **EP 2 658 817 B1**

(12) **FASCICULE DE BREVET EUROPEEN**

(45) Date de publication et mention de la délivrance du brevet:
**07.03.2018 Bulletin 2018/10**

(21) Numéro de dépôt: **11813428.7**

(22) Date de dépôt: **23.12.2011**

(51) Int Cl.:
*C02F 3/28* *(2006.01)*    *C02F 11/04* *(2006.01)*
*C02F 1/74* *(2006.01)*

(86) Numéro de dépôt international:
**PCT/IB2011/055926**

(87) Numéro de publication internationale:
**WO 2012/090139 (05.07.2012 Gazette 2012/27)**

(54) **PROCÉDÉ DE DÉSULFURATION DU DIGESTAT ET DU BIOGAZ D'UN DIGESTEUR, ET INSTALLATION DE PRODUCTION DE BIOGAZ METTANT EN OEUVRE CE PROCÉDÉ**

VERFAHREN ZUR ENTSCHWEFELUNG DER GÄRRESTE UND DES BIOGASES EINES FAULBEHÄLTERS SOWIE DIESES VERFAHREN IMPLEMENTIERENDE ANLAGE ZUR HERSTELLUNG VON BIOGAS

METHOD FOR DESULPHURATION OF THE DIGESTATE AND THE BIOGAS OF A DIGESTER, AND BIOGAS PRODUCTION FACILITY IMPLEMENTING SAID METHOD

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.12.2010 FR 1061265**

(43) Date de publication de la demande:
**06.11.2013 Bulletin 2013/45**

(73) Titulaire: **Degrémont**
**92040 Paris La Défense (FR)**

(72) Inventeurs:
- **BOUCHET, Caroline**
  **F-78580 Maule (FR)**
- **NICOL, Roger**
  **F-92130 Issy-les-Moulineaux (FR)**
- **PREVOT, Claude**
  **F-78140 Vélizy (FR)**

(74) Mandataire: **Cabinet Armengaud Aîné**
**16, rue Gaillon**
**75002 Paris (FR)**

(56) Documents cités:
**EP-A1- 1 970 353       JP-A- 2005 329 377**
**US-A1- 2005 029 189**

EP 2 658 817 B1

## Description

La présente invention concerne un procédé de désulfuration du digestat et du biogaz, c'est à dire élimination des sulfures du digestat et de l'hydrogène sulfuré, ou sulfure d'hydrogène, du biogaz dans un digesteur d'effluents urbains et/ou agricoles et/ou industriels, en voie humide. On entend par effluent tout rejet liquide, pâteux ou solide (eaux résiduaires urbaines ou industrielles, boues issues du traitement d'effluents urbains ou industriels, fraction organique de déchets urbains, industriels ou agricoles).

Le digesteur est constitué d'une enceinte fermée en partie haute, enceinte dans laquelle a lieu une digestion anaérobie d'une masse de produits à traiter. La partie supérieure comporte un volume gazeux d'où est prélevé le biogaz.

Le domaine de l'invention est la digestion anaérobie, ou méthanisation ou fermentation, d'effluents (et/ou résidus et/ou substrats) urbains et/ou agricoles et/ou industriels en voie humide. Cette invention peut s'appliquer sur les installations de traitement des eaux résiduaires municipales et/ou industrielles, les installations du traitement des eaux résiduaires, ou du traitement des ordures ménagères (fraction fermentescible des ordures ménagères et/ou ordures ménagères brutes), et/ou tout type de biodéchets (déchets de restaurants, cantines, invendus de supermarchés, etc.), les installations de traitement de résidus agricoles (déjections animales, résidus végétaux, etc...), les installations de co-digestion (anaérobie) ou co-fermentation ou co-méthanisation où un ou plusieurs substrats sont mélangés, enfin, toute installation produisant du biogaz en voie humide. Dans la suite du texte, il sera parfois utilisé le seul terme « digestion », pour simplifier, à la place de « digestion anaérobie » ou « méthanisation » ou « fermentation » ; il est entendu que le terme « digestion » englobe les termes méthanisation, digestat et/ou fermentation. De même, le digestat correspond à la masse ou au volume global du substrat digéré ou méthanisé ou fermenté.

A l'heure où les préoccupations environnementales deviennent de plus en plus importantes et où les alternatives à la consommation d'énergie fossile sont recherchées, la digestion anaérobie/méthanisation/fermentation d'effluents et/ou de résidus doit être considérée avec beaucoup d'intérêt. Elle permet en effet la production d'un biogaz riche en méthane valorisable en énergie thermique et/ou électrique.

Compte tenu de la composition des effluents et/ou résidus urbains, agricoles ou industriels, le biogaz produit lors de la digestion contient du sulfure d'hydrogène ($H_2S$) dans des teneurs comprises entre 0 et 20 000 ppm (parties par million), selon le traitement appliqué préalablement à la digestion anaérobie/méthanisation/fermentation et selon les substrats/effluents.

Lors de la valorisation thermique et/ou électrique du biogaz - par co(ou tri) génération, chaudière, torchère, piles à combustible, ou indirectement par injection dans le réseau, ou transformation en biogaz carburant - ce sulfure d'hydrogène provoque une corrosion des appareils, et/ou produit par transformation chimique des oxydes de soufre toxiques (SOx) dont les rejets dans l'atmosphère sont réglementés.

On peut également noter que l'efficacité de la digestion peut se trouver limitée en présence d'une quantité très importante d'hydrogène sulfuré, celui-ci ayant un pouvoir toxique vis-à-vis des bactéries de la digestion.

Par ailleurs, la présence de sulfures dissous dans le digestat (en équilibre avec les teneurs en hydrogène sulfuré du biogaz), se traduit par un risque de dégazage ultérieur et des problèmes de sécurité pour les personnes amenées à manipuler/traiter/conditionner le digestat.

Quelle que soit la destination finale de valorisation du biogaz et le devenir du digestat, il s'avère indispensable d'éliminer au moins $H_2S$ dans le biogaz, et de préférence d'éliminer aussi les sulfures du digestat.

Le cycle du soufre est complexe. Dans des conditions anaérobies, la production de sulfures est inévitable. Au pH habituel de fonctionnement des digesteurs (méthaniseurs), une majeure partie des sulfures est désorbée/strippée sous forme d'hydrogène sulfuré dans le biogaz.

Actuellement, l'élimination des sulfures dans le digestat et/ou de l'hydrogène sulfuré dans le biogaz consiste à réaliser des traitements :

- en amont du digesteur : par exemple par l'injection de sels de fer dans le/les substrat(s) et/ou effluents ;
- en aval du digesteur : par un post-traitement du biogaz utilisant des réactions chimiques, physiques, biologiques ou une combinaison de ces procédés élémentaires. Ce post-traitement consiste en un ou plusieurs réacteurs séparés du digesteur et traitant le biogaz après la digestion des effluents/substrats.
- dans le digesteur : par injection de sels de fer ou par micro-aération.

Ces solutions sont généralement onéreuses en coûts d'investissement et en coûts d'exploitation.

Une autre voie utilise l'oxydation biologique des sulfures qui peut produire sélectivement du soufre natif S°, solide jaune amorphe ou non (selon son état de pureté et/ou de cristallisation). C'est le cas notamment lorsque l'accepteur d'électron est l'oxygène et que ce dernier est présent selon des teneurs faibles (inférieures à 0,1 mg/L). Les bactéries thio-oxydantes sont en particulier constituées de bactéries à Gram négatif des espèces *Thiobacillus, Thiomicrospira* et *Thiosphaera.* Certaines bactéries chimiolithotrophes utilisant les sulfures comme donneurs d'électrons sont aussi capables d'utiliser les formes oxydées de l'azote ($NO_2$ et $NO_3$) comme accepteurs d'électrons. La mise en oeuvre de ce type de réaction, sans compromettre les conditions anaérobies favorables à la méthanogénèse est précisément le but de la micro-oxydation.

La micro-oxydation du digesteur est généralement réalisée en méthanisation agricole, avec des digesteurs de capacité réduite correspondant par exemple aux effluents d'une seule exploitation agricole. La micro-oxydation est alors assurée par injection d'air dans le volume gazeux supérieur, également appelé « ciel », du digesteur. La réaction biochimique produit du soufre S° formant des stalactites localisées sous le dôme du digesteur. Les risques principaux liés à cette précipitation sont :

- la détérioration des systèmes de brassage et de reprise du digestat en cas de détachement brutal d'une masse de soufre,
- le risque de dépôt dans toute la ligne biogaz avec détérioration des équipements associés,
- la masse excessive de soufre, sous la coupole ou sous tout autre dispositif de couverture, pouvant provoquer une rupture mécanique.

Ces digesteurs bâchés sont généralement nettoyés une fois par an afin de pallier cet inconvénient. Dans le cadre d'installations à plus grande échelle, il n'est pas envisageable économiquement de vidanger et de nettoyer un digesteur une fois par an. La recommandation classique est une vidange et un curage tous les dix ans.

Enfin, lorsque le digesteur n'est pas brassé au biogaz, le traitement préférentiel du biogaz ne garantit pas une réduction proportionnelle des sulfures du digestat et ne permet pas de prévenir un risque de dégazage ultérieur d'hydrogène sulfuré.

Des digesteurs ont été réalisés avec un brassage au biogaz spécifique afin de permettre la micro-aération au niveau de cannes de brassage. Le risque est d'une part une obturation des cannes de brassage par la précipitation de ce soufre S° avec une diminution de l'efficacité de la digestion à la suite d'une mauvaise homogénéisation, d'autre part dans cette configuration une dissolution de l'oxygène dans le digestat très limitée avec un risque résiduel de formation de dépôts sous le dôme ou la couverture.

Le document US2005/0029189 A1 décrit un digesteur anaérobique dans lequel une micro-aération est effectuée au sein d'une boucle de recirculation interne. Les conditions de la micro-aération ne sont pas spécifiquement détaillées. Les inconvénients des procédés de désulfuration décrits précédemment, proviennent notamment d'une mauvaise dissolution de l'air ou de l'oxydant dans le digestat, et d'une formation de soufre natif dans des zones très localisées, en particulier dans le ciel gazeux des digesteurs. Cette accumulation de soufre a pour effet secondaire une dégradation du matériel du digesteur et/ou de l'efficacité de digestion. Ces procédés ne permettent pas non plus une désulfuration complète du digestat, et les risques d'entraînement (stripping) ultérieur d'hydrogène sulfuré restent importants.

L'invention a pour but, surtout, de fournir un procédé de désulfuration qui ne présente plus ces inconvénients, et qui soit d'une exploitation simple et économique. Il est souhaitable en outre que le procédé puisse être mis en oeuvre aisément sur un digesteur existant. Le procédé de désulfuration du digestat et du biogaz selon l'invention est défini par la revendication 1. De préférence, la quantité d'air ou oxygène injectée dans la boucle de recirculation correspond à celle qui permettrait d'obtenir une concentration équivalente d'oxygène supérieure ou égale à 0.20 X, et de préférence comprise entre 0.60X et 0.20X.

Le temps de contact entre l'oxydant injecté et le digestat depuis le point d'injection jusqu'au point de réintroduction dans l'enceinte est d'au moins 15 secondes.

La vitesse de circulation du digestat dans la boucle est d'au moins 0.6 m/s, de préférence d'au moins 1m/s (1 mètre/seconde).

L'oxydant injecté est de préférence gazeux et constitué par de l'air ou de l'oxygène.

L'invention permet d'obtenir non seulement l'élimination de l'hydrogène sulfuré ou sulfure d'hydrogène du biogaz, mais aussi l'élimination des sulfures du digestat ou substrat.

L'invention est également relative à une installation de production de biogaz telle que décrite dans la revendication 4. La boucle de recirculation peut comporter un bassin de contact, notamment avec moyens d'agitation, pour favoriser un transfert de l'oxydant dans la phase recirculée.

Le point de prélèvement du digestat pour la boucle de recirculation est situé dans la partie inférieure du digesteur, et le point de réinjection du digestat est situé à un niveau de liquide supérieur à celui du prélèvement afin de limiter le dégazage de l'oxydant.

La boucle de recirculation peut comporter au moins un échangeur de chaleur pour réchauffer le digestat et maintenir le digesteur à température constante. L'échangeur de chaleur peut être un échangeur double-enveloppe autour d'une portion de la boucle de recirculation.

L'invention consiste, mises à part les dispositions exposées ci-dessus, en un certain nombre d'autres dispositions dont il sera plus explicitement question ci-après à propos d'exemples de réalisation décrits avec référence aux dessins annexés, mais qui ne sont nullement limitatifs. Sur ces dessins :

Fig. 1 est une coupe axiale verticale schématique d'une installation avec digesteur prévue pour mettre en oeuvre le procédé de désulfuration selon l'invention.

Fig. 2 est une coupe schématique verticale d'une variante de l'installation selon l'invention.

Fig. 3 est une coupe schématique verticale d'une autre variante de réalisation de l'installation selon l'invention.

Fig. 4 est un diagramme illustrant la présence d'oxygène dans le biogaz, portée en ordonnée, en fonction de la concentration de l'oxygène dans la boue recirculée portée en abscisse.

Fig. 5 est un diagramme illustrant la présence d'oxygène dans le biogaz, portée en ordonnée, en fonction de la concentration de l'oxygène dans la boue recirculée portée en abscisse.

Fig. 6 est un diagramme illustrant le dépôt de soufre porté en ordonnée en fonction de la vitesse de recirculation dans la boucle portée en abscisse.

Fig. 7 est une coupe schématique verticale d'une installation selon l'invention avec agitation du digestat par canne de brassage réinjectant du biogaz.

Fig. 8 montre semblablement à Fig. 7 une installation avec agitation mécanique du digestat et

Fig. 9 est une coupe schématique, semblable à Fig. 7, d'une variante avec agitation du digestat par boîtes à bulles de gaz.

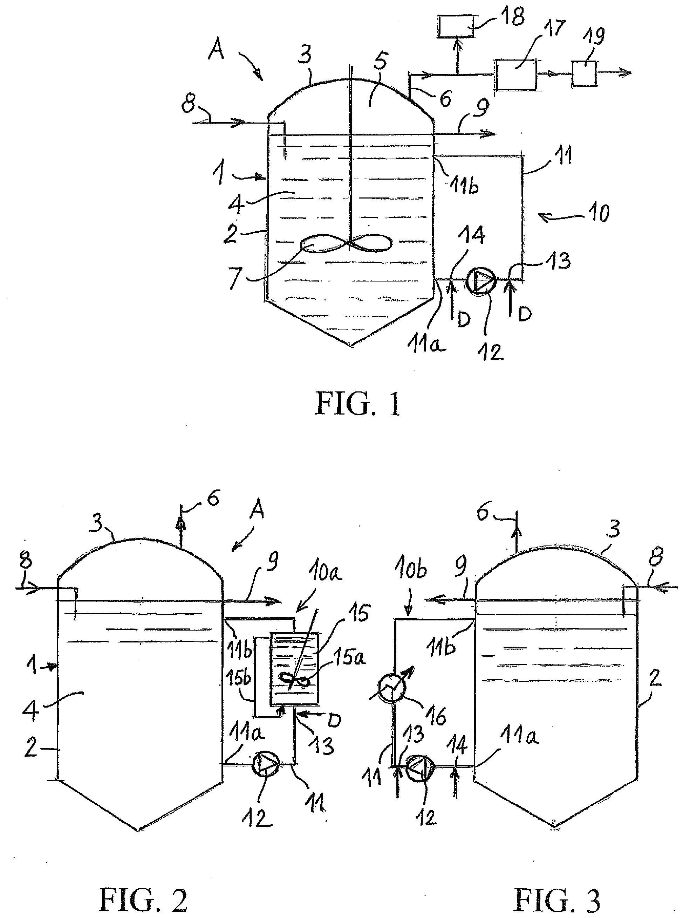

En se reportant à Fig. 1 des dessins, on peut voir une installation A de production de biogaz comprenant un digesteur 1 d'effluents urbains et/ou agricoles et/ou industriels en voie humide. Le digesteur 1 est constitué d'une enceinte 2 fermée, en partie haute, par un dôme 3. Dans l'enceinte 2 a lieu une digestion anaérobie d'une masse de produits à traiter formant un digestat 4 avec un volume gazeux 5, également appelé ciel, au-dessus du digestat d'où est prélevé le biogaz par une conduite 6. Des moyens d'agitation du digestat sont prévus, en particulier des moyens d'agitation mécanique 7 formés par une ou plusieurs hélices supportées par un arbre vertical rotatif.

Les produits à traiter sont introduits dans l'enceinte 2 par une conduite 8 tandis que l'excès de digestat est évacué par une conduite 9 de trop-plein.

L'installation comporte au moins une boucle 10 de recirculation formée par une conduite 11 branchée sur un point de prélèvement 11a situé en partie basse de l'enceinte 2 et un point de réinjection 11b dans l'enceinte située en partie haute.

Une pompe de recirculation 12 est disposée sur la conduite 11 pour faire circuler le digestat depuis le point de prélèvement 11a vers le point de réinjection 11b.

Un dispositif D d'injection d'un oxydant, gazeux ou liquide, est prévu en au moins un point 13, 14 de la boucle de circulation. Le point d'injection 13, selon l'exemple de Fig. 1, se trouve en aval de la pompe 12 tandis que le point d'injection 14 se trouve en amont. Un seul point d'injection ou plus de deux points d'injection peuvent être prévus.

Le diamètre interne de la conduite 11 de la boucle 10 et le débit de la pompe 12 sont choisis pour que la vitesse de circulation du digestat dans la boucle 10 soit suffisante pour empêcher un dépôt de soufre sur les parois des conduites de la boucle 10. Cette vitesse de recirculation du digestat est supérieure à 0,6 m/s (0.6 mètre/seconde), et de préférence supérieure à 0,8 m/s, avantageusement égale ou supérieure à 1 m/s (1 mètre/seconde).

La quantité d'air ou d'oxygène injectée par le ou les dispositifs d'injection D d'oxydant dans la boucle de recirculation correspond à celle qui permettrait d'obtenir une concentration équivalente d'oxygène inférieure ou égale à 0.65X, X étant la concentration de saturation en oxygène dans l'eau pure estimée à la température et pression de la boucle de saturation.

De préférence, cette quantité d'air ou oxygène injectée dans la boucle de recirculation correspond à celle qui permettrait d'obtenir une concentration équivalente d'oxygène supérieure ou égale à 0.20 X, et de préférence comprise entre 0.60X et 0.20X pour assurer un rendement de précipitation du soufre suffisant.

La longueur de conduite de la boucle 10 entre le point d'injection 13, 14 de l'oxydant et le point de réinjection 11b dans l'enceinte 2 est choisie suffisante pour que le temps de contact entre l'oxygène injecté et le digestat recirculé depuis le point d'injection (13, 14) de l'oxydant jusqu'au point de réintroduction (11b) du digestat dans l'enceinte soit d'au moins 15 secondes pour que tout l'oxydant soit dissous dans la phase liquide du digestat avant retour dans l'enceinte 2.

L'invention concerne ainsi un procédé de désulfuration du biogaz par micro-oxydation consistant en une injection d'air, ou d'oxygène ou de tout autre oxydant comprenant une quantité en oxygène appropriée, dans une boucle de recirculation 10 du digestat externe au digesteur. L'injection d'oxydant, air ou oxygène ou tout autre oxydant comprenant une quantité en oxygène appropriée, sous forme gazeuse ou liquide, est avantageusement réalisée au moyen d'un dispositif D diffuseur fines ou moyennes bulles disponible dans le commerce.

La longueur de la conduite 11 est déterminée en ce qu'elle permet un temps de contact d'au moins 15 secondes entre l'oxydant sous forme gazeuse ou liquide et le digestat afin de dissoudre tout l'oxydant dans cette boucle de recirculation. Le diamètre de la conduite 11 est déterminé en fonction d'une vitesse minimale de recirculation de la boue pour limiter les dépôts localisés de soufre natif, ou soufre solide sous la forme $S^0$.

L'invention propose ainsi une micro-oxydation des substrats à digérer dans une boucle de recirculation externe 10 afin d'éliminer les sulfures du digestat et du biogaz et d'éviter les accumulations localisées de soufre. Le soufre formé se trouve alors sous forme particulaire dans la phase liquide ou semi-liquide, et est homogénéisé avec le digestat/substrat grâce au système de brassage 7 du digesteur : il ne peut pas s'accumuler et former des agglomérats nocifs à la digestion. Ce soufre est ensuite évacué avec le digestat/substrat par fournées ou en continu ou en semi-continu, selon le fonctionnement de la digestion.

Selon l'exemple de Fig. 1, une seule boucle de recirculation 10 est représentée mais plusieurs boucles externes pourraient être prévues. La vitesse de recirculation du digestat pour un auto-nettoyage de la conduite 11 est au moins égale à 1 m/s, et de préférence supérieure à cette valeur. L'oxydant gazeux peut être de l'air ou de l'oxygène pur.

Le ou les points de prélèvement du digestat sont situés dans la partie inférieure du digesteur 1 et le digestat est réinjecté en un point 11b à un niveau liquide supérieur à celui du prélèvement 11a. La pression dans la boue est plus élevée au point 11a, près duquel est réalisé l'injection d'oxydant, ce qui est favorable à la dissolution de l'oxydant. En outre, les dépôts éventuels de soufre dans l'enceinte 2 sont aspirés par ce prélèvement en partie basse 11a.

Les éléments des variantes des Fig.2 et 3 identiques ou similaires à des éléments déjà décrits à propos de Fig. 1 sont désignés par les mêmes références, leur description n'étant pas reprise, ou étant effectuée succinctement.

Selon l'exemple de réalisation de Fig. 2, un bassin de contact 15, avec moyens d'agitation 15a, est prévu sur la boucle de recirculation 10a. Le bassin 15 est formé par une capacité insérée dans la boucle, entièrement remplie de digestat sans volume gazeux. Le point d'injection 13 peut se situer juste en aval de l'entrée du bassin 15. Une boucle 15b de recirculation de la fraction de concentrat du bassin 15 peut être également prévue. Le bassin 15 pourrait être prévu sans moyens d'agitation.

Ce bassin 15 facilite le transfert de l'oxydant dans la phase recirculée du digestat. Dans l'exemple de Fig. 2, le bassin 15 se trouve en aval de la pompe 12, mais il pourrait être installé en amont de cette pompe.

Fig. 3 illustre une variante de réalisation selon laquelle la boucle de recirculation 10b intègre un échangeur de chaleur 16 permettant de réchauffer le digestat à l'aide d'un fluide caloporteur, et de maintenir le digesteur à température constante. Le ou les échangeurs 16 peuvent être du type double enveloppe autour d'une portion de la conduite 11 de la boucle de recirculation 10b.

## Exemples de réalisation

Les règles de dissolution de l'oxygène dans l'eau pure sont connues (vitesse de transfert et solubilité maximales) en fonction de la température et de la pression. Cependant, dans des milieux plus concentrés tels que les boues de stations d'épuration industrielles et/ou municipales, déchets liquides et/ou semi-liquides..., ces règles ne sont pas établies : elles dépendent de la concentration, du type de produit.

Des tests de dissolution de l'oxygène/ou de l'air dans différents effluents en vue d'optimiser la désulfuration du biogaz et du digestat à échelle industrielle ont été effectués. Par ailleurs, des tests de désulfuration du digestat ont été réalisés afin de caractériser les vitesses à partir desquelles un risque de dépôt sur la conduite apparaît.

## Essais de laboratoire

Un réacteur de 2,5 m$^3$ a été utilisé pour la digestion. Ce réacteur, du type de celui illustré sur Fig. 1, est maintenu en température par un serpentin (non représenté) placé dans le digesteur, serpentin dans lequel circule de l'eau chaude. Cette eau n'entre pas en contact avec la boue.

L'alimentation du réacteur par la conduite 8 est continue et obtenue à partir d'une pompe péristaltique (non représentée) à débit réglable. La vidange du digesteur est réalisée par un trop-plein 9 qui s'écoule gravitairement dans un bassin de stockage (non représenté).

La boue d'alimentation est prélevée sur une station d'épuration en sortie de l'épaississement. Selon les besoins de l'expérience, cette boue peut être une boue biologique, primaire ou mixte.

Le réacteur 1 est brassé par un moyen mécanique 7 pour la facilité de l'installation. Le temps de séjour de la boue dans le réacteur 1 est variable. Deux conditions de température ont été utilisées :

35°C (conditions mésophiles) avec un temps de séjour de 20 jours,

et 55°C (conditions thermophiles) et 12 jours de temps de séjour. La conduite de recirculation 11 a été définie en ce que le débit de recirculation est de 2,5 m$^3$/j ; il est obtenu à l'aide d'une pompe péristaltique 12 dite pompe de recirculation. Le point d'injection de l'oxydant (de l'air utilisé dance cet exemple) est un point 13 situé en aval de la pompe de recirculation 12. La conduite aval de cette injection est en matière plastique transparente. Plusieurs diamètres de conduites 11 ont été utilisés afin de faire varier la vitesse de recirculation.

Une légère surpression dans le digesteur 1 est assurée par le positionnement d'une cloche à eau 17 située sur la conduite 6 de prélèvement de biogaz. Le biogaz est analysé en continu par un analyseur 18 branché sur la conduite 6 et déterminant les teneurs en CH$_4$ (méthane), CO$_2$ (dioxyde de carbone), H$_2$S (hydrogène sulfuré) et O$_2$ (oxygène).

Une unité d'adsorption 19 peut être prévue sur la conduite 6 en aval de la cloche à eau 17, et le biogaz sortant de l'unité 19 est dirigé vers un brûleur (non représenté). Avantageusement, l'unité 17 est une unité d'adsorption sur charbon actif.

Dans un premier temps, il a été évalué la quantité maximale d'air qui pouvait être dissoute dans la boue recirculée : la présence d'oxygène dans le biogaz, signalée par l'analyseur 18, constituait l'indication d'une dissolution incomplète de l'oxydant. Après avoir établi le débit maximal d'air à injecter, différentes tailles de conduites ont été utilisées afin d'établir la vitesse minimale à appliquer pour éviter ou limiter les dépôts de soufre sur les parois.

*Quantité d'oxygène pouvant être dissoute*

Les essais ont été effectués avec une conduite ayant un diamètre intérieur de 6 mm, et avec une vitesse de recirculation de 1 m/s (1 mètre/seconde). Différentes longueurs de conduites ont été utilisées correspondant à un temps de mise en contact différent.

La concentration de l'oxygène dans la boue, par suite de l'injection d'oxydant, est exprimée en pourcentage de la saturation théorique X de l'oxygène dans l'eau pure dans les conditions de pression et de température de l'essai. Une valeur de 0 % correspond à 0 mg/L d'oxygène dans la boue. Une valeur de 100 % correspond à la concentration X en oxygène dans la boue égale à celle obtenue à saturation dans l'eau, pour une température et une pression données.

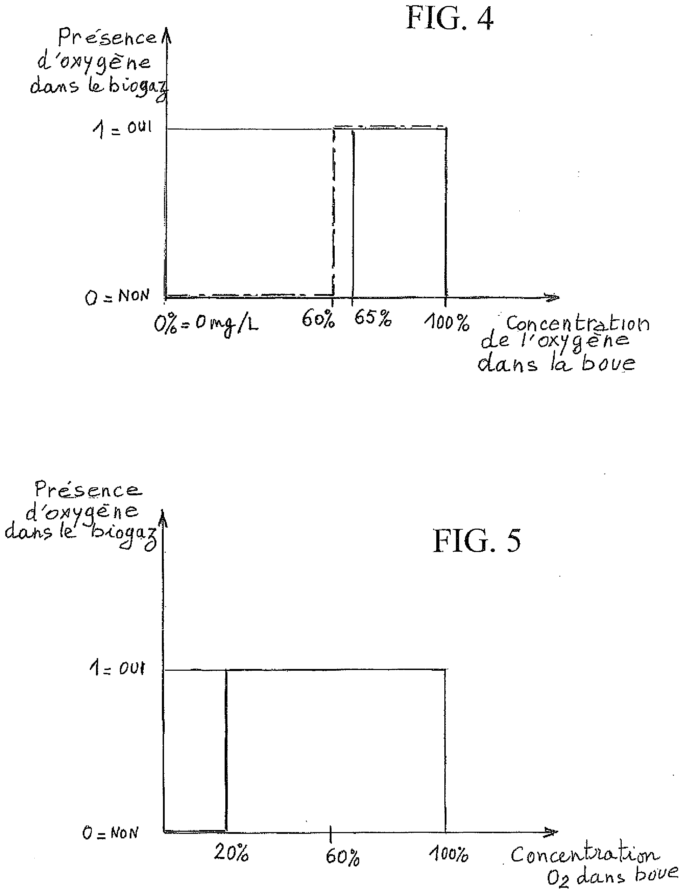

Les résultats pour un temps de contact égal ou supérieur à 15 s sont donnés dans le tableau I ci-après et sont résumés sur le diagramme de Fig. 4. Le temps de contact correspond au temps de parcours de la boue entre le point d'injection de l'oxydant et le point de réinjection dans le digesteur.

*Tableau I*

| Pour un temps de contact supérieur ou égal à 15 s : | | |
|---|---|---|
| Concentration de l'oxygène dans la boue par rapport à la saturation théorique X de l'oxygène dans l'eau | Température de réacteur 35°C | Température de réacteur 55°C |
| 0 % | Absence d'oxygène dans le biogaz | Absence d'oxygène dans le biogaz |
| 10 % | Absence d'oxygène dans le biogaz | Absence d'oxygène dans le biogaz |
| 20 % | Absence d'oxygène dans le biogaz | Absence d'oxygène dans le biogaz |
| 30 % | Absence d'oxygène dans le biogaz | Absence d'oxygène dans le biogaz |
| 40 % | Absence d'oxygène dans le biogaz | Absence d'oxygène dans le biogaz |
| 50 % | Absence d'oxygène dans le biogaz | Absence d'oxygène dans le biogaz |
| 59 % | Absence d'oxygène dans le biogaz | Absence d'oxygène dans le biogaz |
| 60 % | Absence d'oxygène dans le biogaz | *Présence d'oxygène dans le biogaz* |
| 64 % | Absence d'oxygène dans le biogaz | *Présence d'oxygène dans le biogaz* |

(suite)

| Pour un temps de contact supérieur ou égal à 15 s : | | |
|---|---|---|
| Concentration de l'oxygène dans la boue par rapport à la saturation théorique X de l'oxygène dans l'eau | Température de réacteur 35°C | Température de réacteur 55°C |
| 65 % | *Présence d'oxygène dans le biogaz* | *Présence d'oxygène dans le biogaz* |
| 70 % | *Présence d'oxygène dans le biogaz* | *Présence d'oxygène dans le biogaz* |
| 80 % | *Présence d'oxygène dans le biogaz* | *Présence d'oxygène dans le biogaz* |
| 90 % | *Présence d'oxygène dans le biogaz* | *Présence d'oxygène dans le biogaz* |
| 100 % | *Présence d'oxygène dans le biogaz* | *Présence d'oxygène dans le biogaz* |

D'après le tableau I et le diagramme de Fig. 4, il apparaît que pour une température de réacteur, ou digesteur, de 35°C, l'oxygène apparaît dans le biogaz pour une concentration d'oxygène dans la boue égale ou supérieure à 65 % de X.

Pour une température de réacteur de 55°C, l'oxygène est présent dans le biogaz pour une concentration d'oxygène dans la boue égale ou supérieure à 60 % de X.

Sur le diagramme de Fig. 4, la courbe en trait continu correspond à la température de réacteur de 35°C, tandis que la courbe en trait mixte correspond à la température de réacteur de 55°C. Sur cette Fig. 4, l'axe des abscisses correspond aux concentrations d'oxygène dans la boue exprimées en pourcentages de la saturation X en oxygène dans l'eau pure. En ordonnée, l'absence d'oxygène dans le biogaz est représentée par la valeur 0 tandis que la présence d'oxygène est représentée par la valeur 1.

Le tableau II et le diagramme de Fig. 5 correspondent à un temps de contact inférieur à 15 s.

*Tableau II*

| Concentration de l'oxygène dans la boue par rapport à la saturation théorique de l'oxygène dans l'eau | Température de réacteur 35°C | Température De réacteur 55°C |
|---|---|---|
| 0 % à 20 % | Absence d'oxygène dans le biogaz | Absence d'oxygène dans le biogaz |
| 20 % à 100 % | Présence d'oxygène dans le biogaz | Présence d'oxygène dans le biogaz |

Il apparaît clairement que, pour des concentrations d'oxygène dans la boue égales ou supérieures à 20 % de la saturation théorique X de l'oxygène dans l'eau pure, aussi bien à 35°C qu'à 55°C, il y a présence d'oxygène dans le biogaz. Pour des concentrations inférieures, l'oxygène est absent du biogaz.

### Vitesse minimale de recirculation

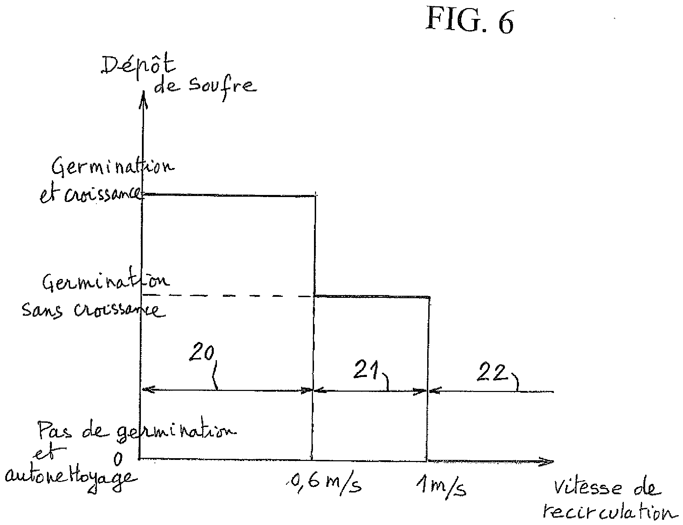

Le diagramme de Fig. 6 résume les résultats des essais effectués. On a estimé la vitesse minimale de recirculation à appliquer pour limiter les dépôts de soufre en utilisant différents diamètres de conduites de recirculation 11. On s'est placé à 50 % de la saturation en oxygène. Les essais ont été réalisés dans les conditions mésophiles (35°C) et thermophiles (55°C).

La boue d'alimentation a été dopée en sulfates afin d'avoir un biogaz très chargé en $H_2S$ permettant une visualisation aisée de la formation de soufre dans la conduite 11. Trois conduites 11, de diamètres différents, ont été utilisées en parallèle afin d'assurer une recirculation totale de 2,5 $m^3$/j de boue à trois vitesses différentes à un temps de séjour de 30 s. Le débit est réparti de manière homogène dans les trois conduites.

Le tableau III ci-après récapitule les observations constatées de la formation de soufre à différentes vitesses de recirculation ; cette formation n'étant pas immédiate, il a été nécessaire d'assurer deux à trois cycles de digestion pour qu'elle apparaisse (acclimatation des bactéries).

***Tableau III***

| Vitesse inférieure à 0,6 m/s | Vitesse supérieure ou égale à 0,6 m/s et inférieure à 1 m/s | Vitesse supérieure ou égale à 1 m/s |
|---|---|---|
| Formation de dépôts de soufre sur la conduite.<br><br>Croissance de ces dépôts. | Formation de particules de soufre sur la paroi.<br><br>Il n'est pas observé de croissance de ces dépôts. L'érosion est continue. | Pas de formation de dépôts sur les parois et auto-nettoyage de la conduite si un dépôt a été formé préalablement.,<br>Il n'est plus observé de dépôts sur ces parois. |

Ces constatations ont été réalisées aux deux températures de digestion 35°C et 55°C.

On a ainsi repéré une zone à risques 20 (Fig. 6) pour des vitesses strictement inférieures à 0,6 m/s où la précipitation de soufre est très importante sur les parois, et les risques de bouchage de conduite sont élevés. Sur Fig. 6, une valeur maximale arbitraire a été portée en ordonnée correspondant à la germination et à la croissance de soufre sur la paroi.

Pour des vitesses comprises entre 0,6 m/s et 1 m/s, des dépôts se forment à la surface mais restent à une épaisseur faible. Cette zone 21 (Fig. 6) est une zone intermédiaire représentée avec une ordonnée arbitraire inférieure à celle de la zone 20 sur Fig. 6 et qui correspond à une germination sur la paroi mais sans croissance. Si des dépôts ont été formés à vitesse plus faible, par exemple dans le cas d'un fonctionnement en mode dégradé de la digestion, les faibles vitesses, inférieures à 1 m/s, ne permettront pas d'éliminer les dépôts une fois le procédé de digestion stabilisé.

Pour des vitesses supérieures à 1 m/s dans la conduite de recirculation 11, il s'agit d'une zone optimale 22 de fonctionnement pour la désulfuration du digestat et du biogaz, sans dépôts de soufre sur les parois.

### *Réalisation industrielle*

Une réalisation industrielle est possible selon le schéma de Fig. 1 combiné le cas échéant avec les variantes de Fig. 2 et 3.

L'invention s'adapte sur un digesteur produisant du biogaz à partir d'un ou plusieurs substrats. La teneur en hydrogène sulfuré du biogaz peut être analysée en continu par l'analyseur adéquat 18 ou par des prélèvements réguliers envoyés à un laboratoire d'analyse, par exemple dans une poche adaptée à ce type de prélèvement.

L'oxydant utilisé dans l'exemple considéré est de l'air comprimé, et on parle alors de micro-aération plutôt que de micro-oxydation. Les débits d'oxydants injectés et de boues recirculées sont de préférence mesurés en continu, mais ceci n'est pas obligatoire.

Une mesure de la pression partielle en oxygène dans le biogaz peut également être prévue, par exemple au niveau de l'analyseur 18, pour le suivi d'un éventuel dysfonctionnement, mais ceci n'est pas indispensable.

Une mesure en continu de la teneur en sulfure du digestat et/ou de la teneur en oxygène dissous du digestat et/ou du potentiel d'oxydoréduction du digestat peut aussi être utilisée pour prévenir tout dysfonctionnement, ou pour servir à des fins de régulation de l'injection d'air ou d'oxydant.

Le dispositif a été mis en place sur une installation de digestion de boues résiduaires d'une station d'épuration urbaine. L'installation de digestion comporte quatre digesteurs de 15 900 m$^3$ chacun, et un gazomètre de 5000 m$^3$.

Le suivi de l'installation avant la mise en place de la micro-aération montre que :

- la teneur en H$_2$S dans le biogaz est de 5000 ppm (parties par million),
- le rendement moyen d'élimination des MV (matières volatiles) est de 50% ;
- la production de biogaz est de 660 Nm$^3$/h/digesteur,
- la production de boues est de 180 m$^3$/h,
- la température de digestion est de 35-37°C,
- les sulfures à éliminer sont issus des sulfates amenés par l'eau brute et du soufre organique, soit une teneur dans les boues de 110 mg/L.

La hauteur d'eau dans le digesteur est de 22 m (22 mètres).

La conduite de recirculation 11 est dimensionnée avec :

- un diamètre de 700 mm,
- une longueur de 40 m entre le point d'injection 13 de l'oxydant dans la boucle 10, et la sortie 11b de la boucle.

La boucle 10 est une boucle de recirculation externe. Le digestat est prélevé en fond de digesteur à un point 11a situé à une hauteur de 2 m au-dessus du radier, c'est-à-dire du fond du digesteur. La boucle de recirculation est réinjectée au point 11b situé au niveau d'une vasque d'alimentation du digesteur, correspondant ainsi au niveau liquide dans le digesteur.

L'injection de l'oxydant, ici de l'oxygène par injection d'air comprimé, a été réalisée de manière progressive (augmentation progressive par paliers), afin de ne pas perturber le système biologique de digestion. L'oxydant est directement injecté à l'aide d'une buse (non représentée) située dans la conduite 11 de recirculation. Le débit injecté est régulé autour d'une valeur de consigne liée à la quantité de sulfures à éliminer.

L'installation a été suivie pendant les quatre mois qui ont suivi la mise en place d'une injection d'air.

Un suivi régulier de la qualité du biogaz et de la boue digérée, ou l'un des dispositifs de régulation précités, permet d'ajuster si nécessaire le débit d'air à injecter si les performances sont modifiées.

Un suivi des paramètres de digestion est également préconisé afin de contrer toute dérive (rendement d'élimination des matières volatiles, élimination de la DCO (Demande Chimique d'Oxygène), concentration en AGV (Acides Gras Volatils), débit de production du biogaz...). Il est également possible de réguler ce débit à injecter autour d'une valeur consigne qui sera définie lors de la mise en route de l'installation.

La stabilisation complète du système est obtenue après au moins deux cycles de digestion (deux fois le temps de séjour hydraulique du substrat ou de l'effluent dans le digesteur).

L'analyse de biogaz est biquotidienne et réalisée avec un analyseur portable de type GA2000 mesurant $CH_4$, $CO_2$, $HO_2$, $H_2S$ et CO (oxyde de carbone).

L'analyse des sulfures du digestat, et de l'alimentation du digesteur, est réalisée de manière hebdomadaire par spectrophotométrie.

Le bilan matière évalue la quantité de sulfures contenus dans le biogaz (équivalent à $H_2S$) auquel sont ajoutés les sulfures retrouvés dans le digestat. L'efficacité du procédé est mesurée sur l'abattement des sulfures par rapport à ce qui arrive en entrée du digesteur.

Les résultats de cet exemple de mise en oeuvre ont été obtenus avec une injection continue d'air dans la boucle de recirculation du digesteur. Cependant, pour des raisons de praticité, il peut être utilisé une injection cadencée d'air. Mais il faudra veiller à injecter de l'air au moins 10 minutes par heure, afin d'entretenir la microbiologie de la réaction. Le débit d'air a été augmenté par paliers.

Les résultats obtenus figurent dans le tableau IV ci-après.

*Tableau IV*

| Durée de l'expérience | Quantité d'air injecté (%) par rapport au dessin de la boucle | Abattement des sulfures (%) <br><br> $1 - \dfrac{\text{Sulfures eq.biogaz} + \text{Sulfures eq.digestat}}{\text{Sulfures entrée digesteur}}$ en % |
|---|---|---|
| T0 (avant installation de l'équipement) | 0 | 0 % |
| T0 à T0 + 7 j | 10 % | 2 % |
| T0 + 7 j à T0 + 14 j | 20 % | 5 % |
| T0 + 14 j à T0 + 21 j | 30 % | 10 % |
| T0 + 21 j à T0 + 28 j | 40 % | 20 % |
| T0 + 28 j à T0 + 35 j | 50 % | 35 % |
| T0 + 35 j à T0 + 42 j | 60 % | 45 % |
| T0 + 42 j à T0 + 49 j | 70 % | 60 % |
| T0 + 49 j à T0 + 56 j | 80 % | 70 % |
| T0 + 56 j à T0 + 63 j | 90 % | 75 % |
| T0 + 63 j à T0 + 70 j | 100 % | 83 % |
| T0 + 100 j | 100 % | 80 % |

Ces résultats ont été obtenus avec une alimentation des digesteurs pendant toute la période des essais. La production de biogaz est restée similaire, à +/-10 % près, et le rendement de digestion (type Van Klick) est également conservé.

Il a également été observé une diminution de la concentration en H$_2$S en entrée de la désodorisation de la station des eaux usées au cours des essais. Cependant, la désodorisation concernant l'ensemble des bâtiments de la station d'épuration, il ne peut pas être mis en évidence l'impact seul de la micro-aération sur la teneur en sulfures de l'air issu du réseau de ventilation de l'ensemble de la station.

L'efficacité du traitement aurait pu être améliorée jusqu'à des valeurs proches de 100 % par une augmentation du débit d'oxydant, mais dans le cadre de cet exemple une efficacité de 80 % était suffisante pour l'abattement des sulfures.

## *Applications industrielles*

Tout digesteur anaérobie ou méthaniseur ou fermenteur en voie humide capable de traiter un ou plusieurs effluents et/ou un ou plusieurs substrats, quelle que soit la température de fonctionnement du procédé, peut faire l'objet de la mise en oeuvre de l'invention.

Le procédé peut être installé par création d'une boucle de recirculation de boues à partir des trous d'homme des appareils de digestion ou méthanisation ou fermentation, ou au niveau de points de prélèvement d'échantillons situés sur le dôme (ou toit) ou en périphérie du digesteur.

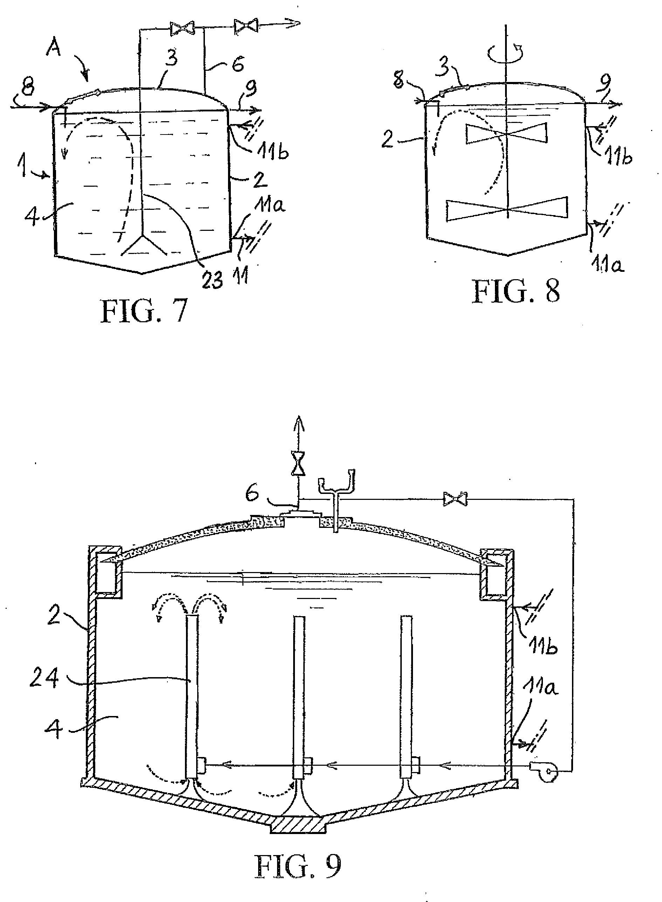

Le brassage du digesteur peut être un brassage mécanique du type à hélice, comme illustré sur Fig. 7, ou autre système de brassage mécanique vertical et/ou horizontal, ou au biogaz avec canne de brassage selon Fig. 8 ou boîte à bulles selon Fig. 9 ou autre, ou hydraulique, avec recirculation, ou autre.

L'invention concerne les installations de méthanisation produisant un biogaz qui peut contenir jusqu'à 20 000 ppm d'H$_2$S et plus particulièrement de 0 à 10 000 ppm, et plus particulièrement encore de 0 à 5000 ppm.

Le biogaz épuré présente une teneur en hydrogène sulfuré au plus égale à 50 ppm, selon les conditions opératoires.

L'invention favorise la dissolution de l'oxygène dans le digestat et empêche l'apparition de stalactites sous le dôme du digesteur, dans le ciel gazeux.

L'invention s'adapte sur tout digesteur en voie humide produisant du biogaz à partir d'un ou plusieurs substrats. La teneur en hydrogène sulfuré du biogaz peut être analysée en continu par un analyseur adéquat ou par des prélèvements réguliers envoyés à un laboratoire d'analyse (par poche spéciale par exemple).

Lorsque l'oxydant utilisé est de l'air comprimé, on parle de micro-aération. Les débits d'oxydant injecté et de biogaz sont préférentiellement mesurés en continu, mais cela n'est pas obligatoire. Une mesure de la pression partielle en oxygène dans le biogaz peut également être prévue pour le suivi d'un éventuel dysfonctionnement.

Une mesure en continu de la teneur en sulfure du digestat et/ou de la teneur en oxygène dissous du digestat et/ou du potentiel d'oxydoréduction du digestat peut aussi être utilisée pour prévenir tout dysfonctionnement ou à des fins de régulation de l'injection d'air ou d'oxydant.

## Revendications

**1.** Procédé de désulfuration du digestat et du biogaz dans une installation de production de biogaz comprenant un digesteur (1) d'effluents urbains et/ou agricoles et/ou industriels, en voie humide, le digesteur étant constitué d'une enceinte (2) fermée en partie haute, dans laquelle a lieu une digestion anaérobie d'une masse de produits à traiter formant le digestat (4), avec un volume gazeux au-dessus du digestat d'où est prélevé le biogaz, le digesteur comportant au moins une boucle de recirculation (10) externe du digestat, **caractérisé en ce que** :

   - le point de prélèvement (11a) du digestat est situé dans la partie inférieure du digesteur (1), et le digestat est réinjecté en un point (11b) à un niveau liquide supérieur à celui du prélèvement (11a),
   - l'on effectue, en au moins un point (13,14) de la boucle de recirculation (10) du digestat , une injection d'un oxydant, gazeux ou liquide, les conditions d'injection étant les suivantes :
   - la vitesse de circulation du digestat dans la boucle (10) est d'au moins 0,6 m/s, de préférence d'au moins 1 m/s pour empêcher le dépôt de soufre sur les parois des conduites de la boucle de recirculation,
   - le temps de contact entre l'oxydant injecté et le digestat recirculé depuis le point d'injection (13,14) de l'oxydant jusqu'au point de réintroduction (11b) du digestat dans l'enceinte est d'au moins 15 secondes pour que tout l'oxydant soit dissous dans la phase liquide du digestat,
   - la quantité en oxygène de l'oxydant injectée dans la boucle de recirculation correspond à celle qui permettrait d'obtenir une concentration équivalente d'oxygène inférieure ou égale à 0,65 X pour éviter la présence d'oxygène dans le biogaz, et correspond à celle qui permettrait d'obtenir une concentration équivalente d'oxygène supérieure ou égale à 0,20 X, pour permettre l'élimination de l'hydrogène sulfuré du biogaz, X étant la concentration de saturation en oxygène dans l'eau pure estimée à la température et pression de la boucle de recirculation.

le soufre produit restant sous forme de particules dispersées dans le digestat et évacuées avec ce dernier.

2. Procédé selon la revendication 1, **caractérisé en ce que** la quantité d'air ou oxygène injectée dans la boucle de recirculation correspond à celle qui permettrait d'obtenir une concentration équivalente d'oxygène comprise entre 0,60 X et 0,20 X, X étant la concentration de saturation en oxygène dans l'eau pure estimée à la température et pression de la boucle de recirculation.

3. Procédé selon l'une quelconque des revendications précédentes, **caractérisé en ce que** l'oxydant injecté est gazeux et constitué par de l'air ou de l'oxygène.

4. Installation de production de biogaz permettant la mise en oeuvre d'un procédé de désulfuration du digestat et du biogaz conforme à l'une des revendications 1 à 3, l'installation comprenant un digesteur (1) d'effluents urbains et/ou agricoles et/ou industriels, en voie humide, le digesteur étant constitué d'une enceinte (2) fermée en partie haute, dans laquelle a lieu une digestion anaérobie d'une masse de produits à traiter formant un digestat (4), avec un volume gazeux (5) au-dessus du digestat d'où est prélevé le biogaz, et au moins une boucle de recirculation (10) externe du digestat entre un point de prélèvement (11a) de l'enceinte et un point de réintroduction (11b), **caractérisée en ce que** :

   - le point de prélèvement (11a) du digestat est situé dans la partie inférieure du digesteur (1), et le digestat est réinjecté en un point (11b) à un niveau liquide supérieur à celui du prélèvement (11a),
   - ladite installation comporte en au moins un point (13,14) de la boucle de recirculation, un dispositif d' injection (D) d'un oxydant, gazeux ou liquide,
   - ladite installation comporte une pompe (12) mettant le digestat en circulation dans la boucle, le diamètre de la canalisation (11) de la boucle et le débit de la pompe (12) étant choisis pour que la vitesse de circulation du digestat dans la boucle (10) soit d'au moins 0,6 m/s, de préférence d'au moins 1 m/s pour empêcher un dépôt de soufre sur les parois des conduites de la boucle de recirculation,
   - la longueur de canalisation de la boucle entre le point d'injection (13,14) et le point de réintroduction (11b) dans l'enceinte est suffisante pour que le temps de contact entre l'oxydant injecté et le digestat recirculé depuis le point d'injection (13,14) de l'oxydant jusqu'au point de réintroduction (11b) du digestat dans l'enceinte soit d'au moins 15 secondes pour que tout l'oxydant soit passé dans la phase liquide du digestat avant retour dans l'enceinte,

5. Installation selon la revendication 4, **caractérisée en ce que** la boucle de recirculation (10) comporte un bassin de contact (15), en particulier avec moyens d'agitation (15a), pour favoriser un transfert de l'oxydant dans la phase recirculée.

6. Installation selon l'une quelconque des revendications 4 ou 5, **caractérisée en ce que** la boucle de recirculation (10) comporte au moins un échangeur de chaleur (16) pour réchauffer le digestat et maintenir le digesteur à température constante.

7. Installation selon la revendication 6, **caractérisée en ce que** l'échangeur de chaleur est un échangeur double-enveloppe autour d'une portion de la boucle de recirculation.

**Patentansprüche**

1. Verfahren zur Entschwefelung des Gärguts und des Biogases in einer Anlage zur Herstellung von Biogas mit einem Faulbehälter (1) für städtische und/oder landwirtschaftliche und/oder industrielle Abwässer im Nassverfahren, wobei der Faulbehälter durch ein im oberen Bereich geschlossenes Gehäuse (2) gebildet ist, in welchem eine anaerobe Vergärung einer zu verarbeitenden Produktmasse, welche das Gärgut (4) bildet, erfolgt, mit einem Gasvolumen über dem Gärgut, aus welchem das Biogas entnommen wird, wobei der Faulbehälter mindestens eine externe Gärgut-Rezirkulationsschleife (10) aufweist, **dadurch gekennzeichnet, dass**:

   - die Gärgutentnahmestelle (11a) sich in dem unteren Bereich des Faulbehälters (1) befindet und das Gärgut an einer Stelle (11b) wieder eingespritzt wird, die bei einem höheren Flüssigkeitspegel als die Entnahmestelle (11a) liegt,
   - an mindestens einer Stelle (13, 14) der Gärgut-Rezirkulationsschleife (10) ein Einspritzen eines gasförmigen oder flüssigen Oxidationsmittels erfolgt, wobei die Einspritzbedingungen wie folgt sind:

- die Zirkulationsgeschwindigkeit des Gärguts in der Schleife (10) beträgt mindestens 0,6 m/s, vorzugsweise mindestens 1 m/s, um das Ablagern von Schwefel an den Wänden der Leitungen der Rezirkulationsschleife zu verhindern,

- die Dauer des Kontakts zwischen dem eingespritzten Oxidationsmittel und dem Gärgut von der Einspritzstelle (13, 14) des Oxidationsmittels bis zu der Stelle (11b) der Wiedereinleitung des Gärguts in das Gehäuse mindestens 15 Sekunden beträgt, so dass das gesamte Oxidationsmittel in der flüssigen Phase des Gärguts gelöst wird,

- die Menge an Sauerstoff des Oxidationsmittels, welche in die Rezirkulationsschleife eingespritzt wird, entspricht derjenigen, die es ermöglichen würde, eine äquivalente Sauerstoffkonzentration zu erhalten, die geringer als oder gleich 0,65 X ist, um das Vorhandensein von Sauerstoff in dem Biogas zu verhindern, und entspricht derjenigen, die es ermöglichen würde, eine äquivalente Sauerstoffkonzentration zu erhalten, die höher als oder gleich 0,20 X ist, um das Eliminieren von Schwefelwasserstoff aus dem Biogas zu ermöglichen, wobei X die geschätzte Sauerstoffsättigungskonzentration in reinem Wasser bei der Temperatur und dem Druck in der Rezirkulationsschleife angibt,

wobei der produzierte Schwefel in Form von Partikeln verbleibt, welche in dem Gärgut verteilt und mit diesem abgeführt werden.

2. Verfahren nach Anspruch 1, **dadurch gekennzeichnet, dass** die Menge an Luft oder Sauerstoff, die in die Rezirkulationsschleife eingespritzt wird, derjenigen entspricht, die es ermöglichen würde, eine äquivalente Sauerstoffkonzentration zwischen 0,60 X und 0,20 X zu erhalten, wobei X die geschätzte Sauerstoffsättigungskonzentration in reinem Wasser bei der Temperatur und dem Druck in der Rezirkulationsschleife angibt.

3. Verfahren nach einem der vorhergehenden Ansprüche, **dadurch gekennzeichnet, dass** das eingespritzte Oxidationsmittel gasförmig ist und durch Luft oder Sauerstoff gebildet ist.

4. Anlage zur Erzeugung von Biogas zur Durchführung eines Verfahrens zur Entschwefelung von Gärgut und von Biogas nach einem der Ansprüche 1 bis 3, wobei die Anlage einen Faulbehälter (1) für städtische und/oder landwirtschaftliche und/oder industrielle Abwässer im Nassverfahren aufweist, wobei der Faulbehälter durch ein im oberen Bereich geschlossenes Gehäuse (2) gebildet ist, in welchem eine anaerobe Vergärung einer zu verarbeitenden Produktmasse, welche das Gärgut (4) bildet, erfolgt, mit einem Gasvolumen (5) über dem Gärgut, aus welchem das Biogas entnommen wird, und mindestens einer externen Gärgut-Rezirkulationsschleife (10) zwischen einer Entnahmestelle (11a) des Gehäuses und einer Wiedereinleitstelle (11b), **dadurch gekennzeichnet, dass**:

- die Gärgutentnahmestelle (11a) sich in dem unteren Bereich des Faulbehälters (1) befindet und das Gärgut an einer Stelle (11b) wieder eingespritzt wird, die bei einem höheren Flüssigkeitspegel als die Entnahmestelle (11a) liegt,

- die Anlage an mindestens einer Stelle (13, 14) der Rezirkulationsschleife (10) eine Einspritzvorrichtung (D) für gasförmiges oder flüssiges Oxidationsmittel aufweist,

- wobei die Anlage eine Pumpe (12) aufweist, welche das Gärgut in der Schleife umwälzt, wobei der Durchmesser der Rohrleitung (11) der Schleife und die Fördermenge der Pumpe (12) derart gewählt sind, dass die Zirkulationsgeschwindigkeit des Gärguts in der Schleife (10) mindestens 0,6 m/s, vorzugsweise mindestens 1 m/s beträgt, um das Ablagern von Schwefel an den Wänden der Leitungen der Rezirkulationsschleife zu verhindern,

- die Länge der Rohrleitung der Schleife zwischen der Einspritzstelle (13, 14) und der Stelle (11b) der Wiedereinleitung in das Gehäuse (3) dazu ausreicht, dass die Dauer des Kontakts zwischen dem eingespritzten Oxidationsmittel und dem Gärgut von der Einspritzstelle (13, 14) des Oxidationsmittels bis zu der Stelle (11b) der Wiedereinleitung des Gärguts mindestens 15 Sekunden beträgt, so dass das gesamte Oxidationsmittel vor der Rückkehr in das Gehäuse in die flüssigen Phase des Gärguts übergegangen ist.

5. Anlage nach Anspruch 4, **dadurch gekennzeichnet, dass** die Rezirkulationsschleife (10) ein Kontaktbecken (15), insbesondere mit Rühreinrichtungen (15a), aufweist, um den Übergang des Oxidationsmittels in die rezirkulierte Phase zu fördern.

6. Anlage nach einem der Ansprüche 4 oder 5, **dadurch gekennzeichnet, dass** die Rezirkulationsschleife (10) mindestens einen Wärmetauscher (16) aufweist, um das Gärgut zu erwärmen und den Faulbehälter auf einer konstanten Temperatur zu halten.

7. Anlage nach Anspruch 6, **dadurch gekennzeichnet, dass** der Wärmetauscher um einen Bereich der Rezirkulationsschleife ein Doppelmantel-Wärmetauscher ist.

## Claims

1. A method for desulfuration of a digestate and a biogas in a digester for urban and/or agricultural and/or industrial effluents, by wet and/or dry route, the digester consisting of a chamber (2) which is closed at the top and in which anaerobic digestion of a mass of products to be treated takes place, forming the digestate (4) with a gas space above the digestate, from which the biogas is withdrawn, the digester comprising at least one external digestate recirculation loop (10), said method being wherein:

   - a digestate withdrawal point (11a) is situated in the lower part of the digester (1), and the digestate is reinjected at a point (11b) at a liquid level higher than the withdrawal point (11a),
   - at at least one point (13, 14) of the digestate recirculation loop (10), a gaseous or liquid oxidant is injected, the injection conditions being as follows:
   - a circulation rate of the digestate in the loop (10) is at least 0.6 m/s, preferably at least 1 m/s, to prevent the deposition of sulfur on the walls of the lines in line in recirculation loop,
   - a contact time between the injected oxidant and the recirculated digestate, from the point (13, 14) of injection of the oxidant to a point of reintroduction (11b) of the digestate in the chamber, is at least 15 seconds for all of the oxidant to be dissolved in a liquid phase of the digestate,
   - the amount of oxygen injected into the recirculation loop corresponds to the amount that would produce an equivalent oxygen concentration of less than or equal to 0.65 X to prevent a presence of oxygen in the biogas, and correspond to the amount that would produce an equivalent oxygen concentration of greater than or equal to 0.20 X to remove hydrogen sulfide from the biogas, where X is the saturation concentration of oxygen in pure water, estimated at the temperature and pressure of the recirculation loop,

   a sulfur produced remaining in the form of particles which are dispersed in the digestate and being removed together with the digestate.

2. The method as claimed in claim 1, wherein the amount of air or oxygen injected into the recirculation loop corresponds to the amount that would produce an equivalent oxygen concentration of between 0.60 X and 0.20 X; where X is the saturation concentration of oxygen in pure water, estimated at the temperature and pressure of the recirculation loop.

3. The method as claimed in claim 1 or 2, wherein the oxidant injected is gaseous and composed of air or oxygen.

4. A plant for producing biogas, for the implementation of a method according to anyone of the claims 1 to 3, the plant comprising a digester (1) for urban and/or agricultural and/or industrial effluents, by wet and/or dry route, the digester consisting of a chamber (2) which is closed at the top and in which anaerobic digestion of a mass of products to be treated takes place, forming a digestate (4), with a gas space (5) above the digestate, from which the biogas is withdrawn, and at least one external digestate recirculation loop (10) between a point of withdrawal (11a) from the chamber and a reintroduction point (11b), wherein:

   - the withdrawal point (11a) of the digestate is situated in the lower part of the digester, and the digestate is reinjected at a point (11b) at a liquid level higher than the withdrawal point,
   - said plant comprises, at at least one point (13, 14) in the recirculation loop, a device (D) for injecting a gaseous or liquid oxidant,
   - said plant includes a pump (12) which circulates the digestate within the loop, the diameter of the piping of the loop and the capacity of the pump being selected such that the circulation rate of the digestate in the loop is at least 0.6 m/s, preferably at least 1 m/s, to prevent deposition of sulfur on the walls of the lines in the recirculation loop,
   - the length of piping in the loop between the injection point (13, 14) and the reintroduction point (11b) in the chamber is sufficient for the contact time between the injected oxidant and the recirculated digestate, from the point (13, 14) of injection of the oxidant to a point of reintroduction (11b) of the digestate in the chamber, is at least 15 seconds, for all of the oxidant to pass into a liquid phase of the digestate before return to the chamber.

5. The plant as claimed in claim 4, wherein the recirculation loop (10) includes a contact basin (15), more particularly with agitating means (15a), to promote transfer of the oxidant into a recirculated phase.

6. The plant as claimed in claim 4 or 5, wherein the recirculation loop (10) includes at least one heat exchanger (16) for reheating the digestate and maintaining the digester at constant temperature.

7. The plant as claimed in claim 6, wherein the heat exchanger is a double-wall exchanger around a portion of the recirculation loop.

FIG. 1

FIG. 2

FIG. 3

FIG. 4

Présence
d'oxygène
dans le biogaz

1 = OUI

0 = NON

0% = 0 mg/L    60%  65%    100%    Concentration
de l'oxygène
dans la boue

Présence
d'oxygène
dans le biogaz

FIG. 5

1 = OUI

0 = NON

20%    60%    100%    Concentration
$O_2$ dans boue

FIG. 6

Dépôt de soufre

Germination et croissance

Germination sans croissance

20

21

22

Pas de germination et autonettoyage

0

0,6 m/s

1 m/s

Vitesse de recirculation

EP 2 658 817 B1

FIG. 7

FIG. 8

FIG. 9

**RÉFÉRENCES CITÉES DANS LA DESCRIPTION**

**Documents brevets cités dans la description**

- US 20050029189 A1